Europäisches Patentamt

European Patent Office

Office européen des brevets

(11) Numéro de publication: **0 353 111**
**A1**

(12) **DEMANDE DE BREVET EUROPEEN**

(21) Numéro de dépôt: **89401783.9**

(22) Date de dépôt: **23.06.89**

(51) Int. Cl.⁵: **C 12 N 15/00**
A 61 K 39/002,
G 01 N 33/569, C 12 Q 1/68,
C 07 K 7/08, C 07 K 15/00

(30) Priorité: **24.06.88 FR 8808535**

(43) Date de publication de la demande:
**31.01.90 Bulletin 90/05**

(84) Etats contractants désignés:
**AT BE CH DE ES FR GB GR IT LI LU NL SE**

(71) Demandeur: **INSTITUT PASTEUR**
28, rue du Docteur Roux
F-75724 Paris Cédex 15 (FR)

**INSTITUT PASTEUR DE LILLE**
1, rue du Professeur Calmette BP 245
F-59019 Lille Cédex (FR)

**INSTITUT NATIONAL DE LA SANTE ET DE LA RECHERCHE MEDICALE (INSERM)**
101, rue de Tolbiac
F-75654 Paris Cédex 13 (FR)

(72) Inventeur: **Capron, André**
58 rue du Capitaine Jasmin
F-59133 Phalempin (FR)

**Pierce, Raymond**
23 rue de Flandre
F-59113 Seclin (FR)

**Tartar, André**
**Rue du Moulin**
F-62490 Vitry en Artois (FR)

**Leite, Paola**
Praia 2 Botasogo
22250 Botasogo Rio de Janeiro (BZ)

**Cesbron-Delauw, Marie-France**
46 rue du Docteur Legay
F-59110 La Madeleine (FR)

**Maes, Pierrette**
11 avenue du roi Albert Premier
F-59290 Wasquehal (FR)

**Darcy, Françoise**
45 rue Vauban
F-59100 Roubaix (FR)

(74) Mandataire: **Orès, Bernard et al**
Cabinet ORES 6, Avenue de Messine
F-75008 Paris (FR)

Le titre de l'invention a été modifié (Directives relatives à l'examen pratiqué à l'OEB, A-III, 7.3)
Le (Les) microorganisme(s) a (ont) été déposé(s) auprès de l'institut Pasteur sous les numéros I-773 et I-876.

(54) **Acide nucléique et/ou ses fragments codant pour la protéine P30 de Toxoplasma gondii, procédés d'obtention et applications.**

㊲ Séquence d'acide nucléique et/ou un fragment de celle-ci codant pour au moins une protéine reconnue par les anticorps anti-P30 de *Toxoplasma gondii* (TG), produits d'expression de cette séquence. L'invention est également relative au procédé d'obtention desdites protéines, à leur application dans le diagnostic précoce, la surveillance et l'immunoprotection de la toxoplasmose.

EP 0 353 111 A1

**Description**

## SEQUENCE D'ACIDE NUCLEIQUE ET/OU UN FRAGMENT DE CELLE-CI CODANT POUR AU MOINS UNE PROTEINE RECONNUE PAR LES ANTICORPS ANTI-P30 DE TOXOPLASMA GONDII, PRODUITS D'EXPRESSION DE CETTE SEQUENCE, LEURS PROCEDES D'OBTENTION ET LEURS APPLICATIONS.

La présente invention est relative à une séquence d'acide nucléique et/ou à un fragment de celle-ci codant pour au moins une protéine reconnue par les anticorps anti-P30 de *Toxoplasma gondii* (TG), ainsi qu'aux produits d'expression de cette séquence. La présente invention est également relative à leurs procédés d'obtention, à leur application dans le diagnostic précoce, la surveillance et l'immunoprotection de la toxoplasmose.

Le protozoaire parasite *Toxoplasma gondii*, membre de la classe des *Sporozoa* et de l'ordre des *Coccidia* est un parasite intracellulaire qui se reproduit dans une grande variété de types de cellules à l'intérieur de ses hôtes, qui sont des mammifères.

Chez l'homme adulte, deux formes du parasite ont été décrites : le "tachyzoïte", qui est la forme multiplicative rencontrée au cours de la phase aiguë de la maladie, et le "bradyzoïte", forme résistante qui persiste enkystée dans les tissus nerveux et est vraisemblablement responsable de l'entretien d'une immunité durable à la réinfection.

Ce parasite est un pathogène important, non seulement chez l'homme mais également en médecine vétérinaire et il est très largement répandu géographiquement, à telle enseigne qu'on a pu établir que près de 500 millions de personnes dans le monde entier présenteraient une réaction sérologique positive à l'infection.

Chez l'homme, la toxoplasmose est souvent asymptomatique, passe la plupart du temps inaperçue et ne présente aucune conséquence. Cependant, dans certains cas, une toxoplasmose peut néanmoins avoir des conséquences graves pour les sujets dits à risque que sont les femmes enceintes et les sujets immunodéprimés. Chez la femme enceinte, une infection toxoplasmique est à l'origine de redoutables complications foetales et néo-natales, si le traitement maternel n'est pas précocement entrepris et poursuivi avec constance. En particulier, la toxoplasmose est responsable d'atteintes oculaires et cérébrales sévères et même mortelles chez les nouveau-nés contaminés par voie transplacentaire ; de telles complications se retrouvent également chez les patients immunodéficients.

L'isolement du parasite n'est possible que dans les formes évolutives ou congénitales ; il est rarement pratiqué et on se contente habituellement du diagnostic sérologique. Les signes cliniques, quand ils existent, sont généralement peu évocateurs, ce qui confère au diagnostic sérologique une valeur essentielle.

La multiplicité des techniques, des antigènes et/ou des anticorps utilisés, rend compte de la difficulté de ce diagnostic, surtout lorsqu'il s'agit de caractériser les immunoglobulines M (IgM), témoins de l'infection récente évolutive.

Certains tests reposent sur la détection des anticorps, éventuellement présents chez un sujet ; d'autres tests reposent sur la détection des antigènes eux-mêmes.

HUGHES, ("CURRENT TOPICS IN MICROBIOLOGY AND IMMUNOLOGY", Vol. 120 (1985), SPRINGER Ed., pages 105-139,) passe en revue les différents tests de sérodiagnostic disponibles : test de coloration de SABIN et FELDMAN, standardisé par BEVERLEY et BEATTIE (1958) et perfectionné par FELDMAN et LAMB (1966), WALDELAND (1976) et BALFOUR et al. (1982), qui requiert des parasites vivants, de grandes quantités de sérum humain normal et des spécialistes bien entraînés pour sa réalisation et son interprétation ; test de détection d'anticorps par immunofluorescence, qui a été appliqué par REMINGTON (1968) et par KARIM et LUDLAM (1975) à la détection d'anticorps IgM, qui peut donner des réactions non-spécifiques (faux positifs) ; les tests d'hémagglutination qui conduisent à une détection trop tardive des anticorps, notamment chez la femme enceinte ; test ELISA pour la détection d'anticorps de *Toxoplasma*, par isolement d'IgM in situ sur la microplaque de test (NAOT et REMINGTON, 1980). En ce qui concerne les tests sérologiques utilisant la détection des antigènes solubles, ces derniers sont obtenus par simple extraction (lyse à l'eau, congélation et décongélation ou éclatement par ultra-sons de tachyzoïtes de *Toxoplasma*). Ces techniques n'assurent la présence d'antigènes de membrane dans le système de test que s'ils sont aisément solubles dans l'eau ou le tampon ou si l'étape finale de centrifugation laisse subsister de petits fragments de membrane en suspension dans la solution.

Les tests qui reposent sur la détection de différents antigènes de *Toxoplasma gondii* par des anticorps appropriés, ont nécessité la production d'anticorps spécifiques de tachyzoïtes.

Il a été montré que la plupart des anticorps monoclonaux dirigés contre la surface des tachyzoïtes (HANDMAN et al., 1980, "Detection and characterization of membrane antigens of *Toxoplasma gondii*", Aust. J. Esp. Biol. Med. Sci., **59**, 303), (SETHI et al., 1980, "Hybridomas secreting monoclonal antibody with specificity for *Toxoplasma gondii*", J. Parasitol., 6, 192), (JOHNSON et al., 1981, "Hybridomas secreting monoclonal antibody to *Toxoplasma gondii*", Aust. J. Exp. Biol. Med. Sci., **59**, 303), (JOHNSON et al., 1983, "Monoclonal antibodies to *Toxoplasma* cell membrane surface antigens protect mice from toxoplasmosis", J. Protozool., **30**, 351), (KASPER et al., 1983, "Purification of a major membrane protein of *Toxoplasma gondii* by immunoabsorption with a monoclonal antibody", J. Immunol., **130**, 2407), (RODRIGUEZ et al., 1985, "Major surface protein of *Toxoplasma gondii* (P30) contains an immunodominant region with repetitive epitopes", Eur. J. Immunol., **15**, 747) reconnaissent une protéine de 27 à 35 kDa, définie par électrophorèse sur gel d'acrylamide (SDS-PAGE) et qu'on a nommée P30 (KASPER, RODRIGUEZ). Des expériences de transfert passif (JOHNSON) ou d'immunisation active avec la P30, partiellement purifiée (ARAUJO et al., 1984, "Partially purified antigen preparations of *Toxoplasma gondii* protect against lethal infection in mice", Infect. Immun., **45**, 122), indiquent de plus que celle-ci joue un rôle dans l'immunoprophylaxie de la toxoplasmose.

Par ailleurs, la présence d'anticorps spécifiques de la P30, dans le sérum de malades présentant une toxoplasmose récente ou lointaine a été démontrée (HANDMAN et al., 1980, "Detection and characterization of membrane antigens of *Toxoplasma gondii*", J. Immunol., **124**, 2578), (KASPER et al.), (SHARMA et al., 1983, "Western blot analysis of the antigens of *Toxoplasma gondii* recognized by human IgM and IgG antibodies", J. Immunol., **131**, 977).

Comme exemple de détection des anticorps anti-TG, on peut citer le test immunoenzymatique décrit dans l'article de CESBRON & al., publié en 1986 ; il s'agit d'une méthode de capture des IgM anti-P30. Le principe du test est basé sur la capture des IgM sériques que l'on révèle indirectement, en deux temps, par un couple antigène/anticorps monoclonal dirigé contre la P30. Ce test améliore déjà la sensibilité du diagnostic et est nettement supérieur aux tests précédemment cités et notamment à l'immunofluorescence ou l'hémagglutination.

Aussi bien les tests reposant sur la détection des antigènes de TG par des anticorps spécifiques, que les tests reposant sur la détection des anticorps anti-TG produits par le sujet, disponibles à l'heure actuelle, ne présentent pas les caractéristiques de fiabilité recherchée, dans tous les cas de toxoplasmose.

Les Inventeurs ont, en conséquence, considéré comme indispensable de mettre au point des tests qui puissent avantageusement être fiables, quelque soient le type et le stade de la toxoplasmose et ainsi d'améliorer encore la spécificité et la sensibilité de détection de la toxoplasmose, notamment évolutive, et d'éviter ainsi les faux négatifs et les faux positifs.

Les Inventeurs se sont, en conséquence donné pour but de pourvoir, notamment, à des produits de diagnostic qui peuvent être mis en oeuvre dans un procédé de dia gnostic, qui ne présente pas les inconvénients des procédés précédemment proposés et notamment en ce qu'ils sont indépendants de l'état du *Toxoplasma gondii*.

La présente invention a pour objet une séquence d'acide nucléique, caractérisée en ce qu'elle présente la séquence en bases nucléotidiques (I) suivante :

```
1        10          20          30          40          50
AAGGGGAGACCGGTGGACGGTCCAGGTCTTCGTGGAATGCTCGTCTTGCGCGCACCGGC

60        70          80          90          100         110
CGAAATTTCCGGTTCTACGACCCGGCGAAAGTGAAGAGCCTCGTGGTCACGGACTTCAG

120       130         140         150         160
TCGCCTCATGCTCCGCAAGGCCCTGGAGAAGAAAGAAGCCCTCAAG        (I),
```

et en ce qu'elle code pour une protéine reconnue par les anticorps anti-P30.

Cette séquence a été dénommée clone P-30.8 par les Inventeurs.

La présente invention a également pour objet une séquence d'acide nucléique, caractérisée en ce qu'elle comprend au moins un fragment de la séquence de formule (I) ci-dessus et en ce qu'elle présente la séquence en bases nucléotidiques de formule (II) ci-après :

```
10        20        30        40        50        60
GTATCAGACCCTCGTCAAAAGTATCGTTCTTCAGCTGCGATAGAAGCGTCGCAGTGACGT

70        80        90        100       110       120
CAAGGAGCACAGTGGCCAAGCTATACACACAGTCGTACCTTAAGTGACGATGCATTTTCT

130       140       150       160       170       180
CGAGTTTCTCGCCAACACTCCGGTTGCTTGAGAAGGGGAGACCGGTGGACGGTCCAGGTC

190       200       210       220       230       240
TTCGTGGAATGCTCGTCTTGCGCGTAAGCAAGCGATCGACGACGGGCAACAAGAGGAACG
```

```
        250         260         270         280         290         300
         ↓           ↓           ↓           ↓           ↓           ↓
AGGTCAGCTTCTTCAGTGCCGTTCAATCGGTATACGTCCAGCTGGGTCGCTGGAGTCGAG

        310         320         330         340         350         360
         ↓           ↓           ↓           ↓           ↓           ↓
ACGCGGTTCGCAAGAGAGCGGACTGCGTGCATCTGTTGGGTTCGACAGTCGTGTGCTTCG

        370         380         390         400
         ↓           ↓           ↓           ↓
ATGTCGAAAGGGGCAGCTGCGAGTCGGGGAGTTTCGTCGACCTGCAG    .
```

(II).

Cette séquence est dénommée clone GPP5 par les Inventeurs et correspond à un fragment d'un insert de 1600 paires de bases dénommé G42 par les Inventeurs et correspondant à un fragment de l'ADN génomique de *Toxoplasma gondii*.

On entend par séquence d'acide nucléique, dans la présente invention, aussi bien une séquence d'ADN double brin, une séquence d'ADN simple brin que les produits de transcription desdites séquences d'ADN.

La présente invention a également pour objet des oligonucléotides, caractérisés en ce qu'ils sont constitués par un fragment d'une séquence nucléotidique conforme à l'invention.

Parmi ces fragments, on peut citer en particulier :

- un oligonucléotide, caractérisé en ce qu'il présente la séquence de formule III suivante :

5′ CAGCTGCGATAGAAGCGTCGCAGTG    (III)

Une telle séquence correspond en particulier à la séquence de bases 32-56 de la séquence de formule II ;

- un oligonucléotide, caractérisé en ce qu'il présente la séquence de formule IV suivante :

5′ GGGGAGACCGGTGGACGGTCCAGGTC    (IV)

Une telle séquence correspond en particulier à la séquence de bases 155-180 de la séquence de formule II ou à la séquence de bases 3-28 de la séquence de formule I ;

- un oligonucléotide, caractérisé en ce qu'il présente la séquence de formule (V) suivante :

5′GCAGGTCGACCCAGCGACCTCAGC    (V)

Une telle séquence correspond en particulier à la séquence de bases 275-298 de la séquence de formule II.

Selon un mode de réalisation avantageux, lesdits oligonucléotides sont obtenus par synthèse.

La présente invention a également pour objet des paires d'amorces pour la synthèse d'un acide nucléique de *Toxoplasma gondii*, caractérisées en ce que chaque amorce comprend une séquence ou un fragment de séquence nucléotidique telle que définie ci-dessus.

De telles amorces permettent la synthèse spécifique de l'ADN ou de l'ARN de *Toxoplasma gondii*.

Conformément à l'invention, lesdistes amorces peuvent avantageusement être marquées par tout moyen approprié (biotinylation).

Selon un mode de réalisation avantageux desdites paires, elles sont constituées par un oligonucléotide de formule III apparié à un oligonucléotide de formule V, pour la synthèse dudit acide nucléique.

La présente invention a également pour objet des sondes nucléotidiques, caractérisées en ce qu'elles comprennent une séquence nucléotidique ou un fragment de celle-ci telle que définie ci-dessus, éventuellement marquée à l'aide d'un marqueur tel qu'un isotope radioactif, une enzyme appropriée, un fluorochrome ou par toute autre modification chimique ou un anticorps.

Selon un mode de réalisation avantageux de l'invention, ladite sonde est un oligonucléotide de formule IV ci-dessus.

La présente invention a également pour objet une protéine et/ou un fragment de celle-ci présentant des propriétés antigéniques, caractérisée en ce qu'elle est re connue par les anticorps anti-P30 et en ce que sa séquence en acides aminés (VI), déduite de la séquence (I) ci-dessus, est la suivante :

Gly$^1$-Glu-Thr-Gly-Gly-Arg-Ser-Arg-Ser-Ser$^{10}$-Trp-Asn-Ala-Arg-Leu-Ala-Arg-Thr-Gly-Arg$^{20}$-Asn-Phe-Arg-Phe-Tyr-Asp-Pro-Ala-Lys-Val$^{30}$-Lys-Ser-Leu-Val-Val$^{35}$-Thr-Asp-Phe-Ser-Arg$^{40}$-Leu-Met-Leu-Arg-Lys-Ala-Leu-Glu-Lys-Lys$^{50}$-Glu-Ala-Leu-Arg    (VI)

La présente invention a également pour objet un peptide, caractérisé en ce qu'il présente la séquence (VII) suivante en acides aminés :

Thr-Asp-Phe-Ser-Arg-Leu-Met-Leu-Arg-Lys-Ala-Leu-Glu-Lys-Lys-Glu-Ala-Leu-Arg,    (VII)

en ce qu'il est reconnu par les anticorps anti-P30 et en ce qu'il correspond à la fraction C-terminale de la protéine de formule VI ci-dessus.

Selon une variante du peptide de formule VII, l'acide aminé N-terminal est une acétylcystéine et ledit peptide présente la séquence (VIII) suivante en acides aminés :

AcétylCys-Asp-Phe-Ser-Arg-Leu-Met-Leu-Arg-Lys-Ala-Leu-Glu-Lys-Lys-Glu-Ala-Leu-Arg.    (VIII)

La présente invention a également pour objet un conjugué présentant des propriétés antigéniques, caractérisé en ce qu'il est constitué par un peptide de formule VII ou de formule VIII, couplé par covalence à une protéine de transport.

Selon une disposition avantageuse de ce mode de réalisation, ladite protéine de transport est constituée par de l'albumine bovine.

La présente invention a également pour objet une protéine et/ou un fragment de celle-ci présentant des propriétés antigèniques, caractérisée en ce qu'elle est reconnue par les anticorps anti-P30 et en ce que sa séquence en acides aminés (IX), déduite de la séquence II ci-dessus est la suivante :

Glu-Gly-Glu-Thr-Gly-Gly-Arg-Ser-Arg-Ser-Ser-Trp-Asn-Ala-Arg-Leu-Ala-Arg-Lys-Gln-Ala-Ile-Asp-Asp-Gly-Gln-Gln-Glu-Glu-Arg-Gly-Gln-Leu-Leu-Gln-Cys-Arg-Ser-Ile-Gly-Ile-Arg-Pro-Ala-Gly-Ser-Leu-Glu-Ser-Arg-Arg-Gly-Ser-Gln-Glu-Ser-Gly-Leu-ArgAla-Ser-Val-Gly-Phe-Asp-Ser-Arg-Val-Leu-Arg-Cys-Arg-Lys-Gly-Gln-Leu-Arg-Val-Gly-Glu-Phe-Arg-Arg-Pro-Ala.    (IX)

La présente invention a également pour objet un produit d'expression de la protéine de formule (VI), caractérisé en ce qu'il est exprimé par un vecteur d'expression recombinant dans lequel a été insérée la séquence d'ADN de formule I et en ce qu'il a un poids moléculaire de 120 KDa.

Selon une disposition avantageuse de ce mode de réalisation, ledit produit d'expression comprend un fragment peptidique de 6 KDa qui correspond à une partie d'une protéine antigénique de *Toxoplasma gondii*, est identique à la protéine de formule (VI) , ci-dessus, et porte au moins un épitope reconnu par les anticorps anti-P30.

La préparation de la protéine et/ou des peptides et/ou des conjugués, conforme(s) à l'invention, est réalisée de manière connue par synthèse en phase solide, selon la méthode de MERRIFIELD.

La présente invention a également pour objet un plasmide recombinant comportant un insert d'ADN de formule I ci-dessus.

Ledit plasmide recombinant est déposé auprès de la Collection Nationale de Cultures de Micro-organismes tenue par l'INSTITUT PASTEUR, en date du 24 juin 1988 sous le n° I-773.

La présente invention a également pour objet un plasmide recombinant comportant un insert d'ADN contenant le fragment d'ADN de formule II ci-dessus.

Cet insert d'ADN est le fragment de 1600 paires de bases de l'ADN génomique de *Toxoplasma gondii* dénommé G42 par les Inventeurs.

Ledit plasmide recombinant est déposé auprès de la Collection Nationale de Cultures de Micro-organismes tenue par l'INSTITUT PASTEUR, en date du 22 juin 1989 sous le n° I-876.

La présente invention a, de plus, pour objet des produits de diagnostic de la toxoplasmose, caractérisés en ce qu'ils contiennent en tant que constituants actifs, une protéine de formule VI et/ou une protéine de formule IX et/ou un peptide de formule VII ou VIII et/ou un conjugué conforme(s) à l'invention reconnu(s) par les anticorps anti-P30.

La présente invention a également pour objet des produits de diagnostic de la toxoplasmose, caractérisés en ce qu'ils contiennent comme constituants actifs, une séquence d'acide nucléique et/ou un fragment de celle-ci conforme à l'invention.

La présente invention a, de plus, pour objet des vaccins propres à procurer une protection significative contre la toxoplasmose, caractérisés en ce qu'ils contiennent en tant que constituant actif une protéine et/ou un peptide et/ou un conjugué conforme(s) à l'invention, éventuellement associé à au moins un véhicule pharmaceutique approprié.

La présente invention a également pour objet un procédé de détection et/ou de surveillance de la toxoplasmose, notamment de la toxoplasmose aiguë et de la toxoplasmose congénitale, caractérisée en ce qu'elle met en oeuvre au moins un produit de diagnostic conforme à l'invention.

Selon un mode de réalisation, on met en contact les produits de diagnostic représentés par les protéine(s) et/ou peptide (s) et/ou conjugué(s) présentant une activité antigénique telle que définie plus haut, ci-après dénommés fractions antigéniques, avec un échantillon biologique à tester, déterminant ainsi la liaison desdites fractions antigéniques avec des anticorps anti-P30, dans le cas où ceux-ci sont présents dans l'échantillon biologique à tester, et en ce que ledit complexe est révélé de manière appropriée.

Selon un autre mode de réalisation, on met en contact des produits de diagnostic représentés par une sonde conforme à l'invention, avec un échantillon biologique traité de manière appropriée, de façon à permettre l'accès de leurs acides nucléiques à la sonde et réalisation d'une hybridation effective lorsque les toxoplasmes recherchés sont présents, élimination de toute sonde non hybridée et détection appropriée des sondes d'ADN retenues par hybridation.

Selon une disposition avantageuse de ce mode de réalisation, préalablement à l'hybridation, on met en contact, dans des conditions appropriées, l'acide nucléique à détecter avec une paire d'amorces conformes à l'invention, pour amplifier au moins un fragment dudit acide nucléique.

L'amplification mise en oeuvre est notamment l'une des techniques d'amplification génétiques telles que la méthode dite Qβ réplicase (LIZZARDI PM. et al., Biotechnol., 1988, 6) ou la méthode dite réaction de polymérisation en chaîne (P.C.R.) décrite dans les Demandes de brevets européens n° 200 363, n° 201 184 et n° 229 701 déposés par CETUS CO.

Un tel test diagnostique a l'avantage de permettre la détection spécifique de l'ADN génomique de *Toxoplasma gondii* dans des prélèvements biologiques ou des échantillons tissulaires.

Dans le cas de la toxoplasmose congénitale, il s'agira du sang de cordon ou de tissu placentaire ; pour les malades immunodéprimés, il s'agira de lavages bronchoalvéolaires, de liquide céphalorachidien ou du sang.

La présente invention a de plus pour objet un kit, ou coffret pour la détection de *Toxoplasma gondii* dans des échantillons biologiques, caractérisé en ce qu'il com prend :
- une quantité appropriée de protéine et/ou de peptide et/ou de conjugué, conformes à l'invention, le cas échéant un témoin positif, un témoin négatif et tous autres réactifs appropriés.

En variante, le kit comprend une quantité appropriée de sonde conforme à l'invention, éventuellement associée à une quantité appropriée d'au moins une paire d'amorces convenables et le cas échéant des préparations témoins d'acides nucléiques de *Toxoplasma gondii* et tous autres réactifs appropriés.

Outre les dispositions qui précèdent, l'invention comprend encore d'autres dispositions qui ressortiront de la description qui va suivre, qui se réfère à des exemples de clonage du gène codant pour la P30 et à l'utilisation de la P30 comme vaccin.

Il doit être bien entendu, toutefois, que ces exemples sont donnés uniquement à titre d'illustration de l'objet de l'invention, dont ils ne constituent en aucune manière une limitation.

**EXEMPLE 1 : PURIFICATION DE L'ANTIGENE DE SURFACE RECONNU PAR LES ANTICORPS ANTI-P30 :**

a) Protocole d'extraction des antigènes membranaires des tachyzoïtes :

La souche virulente RH de *Toxoplasma gondii* est maintenue par des passages bihebdomadaires dans la souris SWISS. Les parasites sont récoltés à partir du péritoine des souris infectées trois jours auparavant. Ils sont filtrés sur filtres de polycarbonate, 3 μm (Nuclepore) et lavés trois fois avec du PBS (tampon phosphate de sodium 10 mM pH 7,2, NaCl 150 mM). Le culot final est remis en suspension avec du PBS contenant 0,5 % de Nonidet NP40 (Sigma) et 100 unités/ml d'aprotinine (Sigma). Après une incubation pendant une heure à 4°C, l'extrait est centrifugé à 10000 g pendant vingt minutes à 4°C. Le surnageant est utilisé par la suite comme source d'antigène reconnu par des anticorps anti-P30.

b) Purification de l'antigène reconnu par des anticorps anti-P30 par HPLC :

L'extrait total de tachyzoïtes (500 μl) est injecté sur une colonne Vydac $C_{18}$ (4,6 x 250 mm) avec une porosité de 300 Å et des microbilles de 5 μm. L'élution est effectuée avec un mélange $CH_3CN/H_2O/CF_3COOH$ (5/95/0,05 par volume) pendant 12 minutes suivie d'un gradient linéaire pendant 180 minutes jusqu'à 80 % en $CH_3CN$, cette concentration étant maintenue pendant 5 minutes. Le pic contenant l'antigène reconnu par les anticorps anti-P30 est élué à une concentration en $CH_3CN$ de 34,4 % après 90 minutes.

c) Purification de l'antigène reconnu par les anticorps anti-P30 par chromatographie d'immunoaffinité :

L'antigène reconnu par les anticorps anti-P30 a été purifié exactement comme décrit par Santoro et al (1985). L'anticorps monoclonal (mAb) est fixé au support d'affinité activé par le glutaraldéhyde (Boehringer, Mannheim, R.F.A.) suivant les recommandations du fabricant. Brièvement, l'adsorbant d'affinité (1g) est remis en suspension dans du NaCl 0,9 % (5ml) contenant le mAb à 20 mg/ml. Après une incubation de 4 heures à température ambiante avec agitation, le gel est transvasé dans une colonne chromatographique et lavé avec du NaCl 1,5 %, jusqu'à ce que la densité optique (D.O.) de l'éluat soit inférieure à 0,005. Les groupements aldéhydes libres résiduels sont saturés avec de l'éthanolamine-HCl 0,3M (pH 7,5) pendant une heure. Après des lavages successifs avec 50 ml de NaCl 0,9 %, 50 ml de glycine 0,5 M, pH 3,75 et 100 ml NaCl 0,9 %, l'immunoadsorbant est stocké à 4°C.

La purification de la P30 est effectuée pendant une nuit à 4°C en faisant circuler 10 ml d'un extrait total de tachyzoïtes de *Toxoplasma gondii* (200 mg) sur la colonne d'immunoadsorbant en circuit fermé avec un débit de 2 ml/heure. Le système chromatographique est ensuite ouvert et la colonne est lavée avec du NaCl 0,9 %. L'antigène lié spécifiquement à l'immunoadsorbant est élué à l'aide de la triéthylamine 0,1 M (pH 11,5) et neutralisé rapidement avec du HCl 1M. Après une dialyse exhaustive contre du PBS, pH 7,2, la P30 pure est stockée à -70°C.

**EXEMPLE 2 : PREPARATION DES PRODUITS DE TRADUCTION DES ARNs MESSAGERS DE TACHYZOITE :**

a) Extraction de l'ARN total :

On utilise la méthode d'AUFFRAY et ROUGEON (Eur. J. Biochem, 1980, 107, 303). Les tachyzoïtes sont lysés par addition d'une solution de LiCl 3M, Héparine 200 μg/ml, acétate de sodium 10 mM pH 5, SDS 0,1 %.

Après homogénéisation au Potter et conservation toute la nuit pour précipiter les ARNs, le lysat est centrifugé à 16300 g pendant 40 minutes, à 4°C. Le culot est lavé deux fois par remise en suspension dans une solution de LiCl 4M, Urée 8M puis de nouveau centrifugé à 16300 g pendant 40 minutes, à 4°C. Le culot est ensuite dissous dans de l'eau, la solution est amenée à 0,1M en acétate de sodium pH 5 et à 0,1 % en SDS et extraite successivement avec un volume de phénol saturé en eau et par un volume de phénol-chloroforme. La phase aqueuse est finalement amenée à 0,2 M en acétate de sodium pH 5, puis l'ARN est précipité par 2,5 volumes d'éthanol à -20°C pendant une nuit.

b) Purification des ARNs messagers :

On utilise la méthode d'ARNEMANN et al. (Nucl. Acids Res., 1977, 4, 4023-4026). Cette méthode consiste à séparer les ARNs poly A+ de l'ARN total par deux chromatographies successives sur oligo (dT) cellulose.

**EXEMPLE 3 : CLONAGE DES PRODUITS D'EXPRESSION :**

a) Préparation de la banque de cDNA à partir de l'ARNm de tachyzoïtes :

La synthèse du cDNA est réalisée selon la méthode de GUBLER ET HOFFMANN (Gene, 1983, 25, 263). Le premier brin d'ADN complémentaire est synthétisé par l'action de la transcriptase réverse de AMV. L'ADN, rendu double brin par l'action conjointe de la RNAse H et de la polymé rase I, est ensuite traité à la T4 DNA polymérase pour rendre ses extrémités franches. Après méthylation par l'enzyme Eco RI méthylase, on ajoute des "linkers" Eco RI aux extrémités des cDNAs par l'action de la T4 DNA ligase. Les cDNAs sont ensuite digérés par l'enzyme Eco RI et sont purifiés par chromatographie sur une colonne AcA 34 Ultrogel (0,2 x 30 cm) selon la méthode de WATSON ET JACKSON (in Glover D.M. éd. : DNA Cloning, vol. I.A practical approach : Oxford, Washington IRL Press, p. 49, 1985). On insère ensuite les cDNAs (500 à 7000 paires de bases) dans le site EcoRI du phage lambda gt11 (Huynh et al., 1985, in Glover D.M. éd. : DNA Cloning, vol. I.A practical approach : Oxford, Washington IRL Press, p. 49). Après ligation, les phages recombinants sont empaquetés in vitro. Une banque de 3 x 10$^6$ phages est ainsi constituée.

b) Criblage de la banque et sélection des candidats "P30" :

Le dérivé du phage lambda gt11 permet l'expression de gènes étrangers sous le contrôle du promoteur Lac, après fusion du cDNA avec le gène de la β-galactosidase de E. coli (Young et Davis, 1983).

Un échantillon de la banque en lambda gt11 est inoculé sur la souche de E. coli Y 1090 à une dilution représentant 10$^4$ phages par boîte de 90 cm de diamètre. L'induction de l'expression de la protéine sous contrôle du promoteur Lac est déclenchée à 42°C en présence d'isopropyl thio-β-galactoside (IPTG). Les protéines synthétisées sont adsorbées sur filtre de nitrocellulose pour être incubées en présence d'anticorps de lapin anti-P30, conformément à l'exemple 1. Les anticorps fixés sont détectés par un second anticorps anti-IgG de lapin marqué à la peroxydase ; ce complexe est ensuite révélé par une coloration avec le 4-chloro-1-naphthol en présente de H$_2$O$_2$. Cette détection permet d'identifier les phages recombinants dont l'insertion de cDNA dirige la synthèse d'une proteine ou fraction de protéine portant les épitopes reconnus par les anticorps anti-P30.

## EXEMPLE 4 : SOUS-CLONAGE DANS LE PLASMIDE pUC 13 ET LE PHAGE M13 mp10 :

Après purification du clone P30-8, les phages sont produits en grandes quantités, purifiés et l'ADN extrait suivant des techniques classiques (Maniatis et al, 1982). L'ADN est ensuite hydrolysé par l'enzyme Eco RI, et l'insert séparé par électrophorèse sur un gel d'agarose 2 % avant d'être récupéré par électroélution. L'insert est sous-cloné dans le site Eco RI du plasmide pUC 13 (Pharmacia) par action de la T4 DNA ligase et le plasmide recombinant obtenu, conforme à l'invention, est utilisé pour transformer la souche E. coli JM 109. Le plasmide recombinant est préparé en grande quantité par la méthode de Birnboim et Doly (1979) et l'insert repréparé après hydrolyse par Eco RI comme décrit précédemment. Le sous-clonage dans le vecteur phagique M13 mp10 est effectué suivant les instructions du fournisseur (Amersham) et l'ADN de phage simple brin utilisé pour les déterminations de séquence par la méthode de Sanger et al.

## EXEMPLE 5 : SYNTHESE DE PEPTIDES :

Le peptide correspondant à la séquence C-terminale du clone est synthétisé en phase solide (Merrifield, 1963) et les groupements actifs protégés. Après clivage avec du HF, le peptide est purifié par gel filtration sur Fractogel TSK-HWUO-S (Merck) en présence de HCl pH2, puis lyophilisé. La pureté du peptide est vérifiée par chromatographie en couche mince et par HPLC en phase inverse. L'identité du peptide est vérifiée par analyse des acides aminés après hydrolyse acide.

Le peptide est conjugué à l'albumine bovine avec du glutaraldéhyde : le peptide (1,5 μmole) est dissous avec la protéine porteuse (2mg) dans un tampon phosphate de sodium pH7 et le pH est ensuite ajusté à 8 avec du bicarbonate de sodium. Une solution de glutaraldéhyde à 2,5 % (20 μl) est ajoutée quotidiennement pendant trois jours, sous agitation constante. Après quatre jours, le mélange est dialysé contre du NaCl 0,15 M.

## EXEMPLE 6 : PROPRIETE VACCINANTE DE L'ANTIGENE P30 :

L'immunisation de lapins avec 150 μg de l'antigène reconnu par les anticorps anti-P30 purifié soit par immunoadsorption soit par HPLC est réalisée selon la technique de Vaitukatis et al. (1971). Une injection de rappel de 30 μg est effectuée un mois plus tard par voie intramusculaire.

## EXEMPLE 7 : DEPISTAGE ET IDENTIFICATION DE *TOXOPLASMA GONDII* PAR LA PCR :

On utilise les amorces 1 et 2 conformes à l'invention dans la technique PCR et on effectue 30 cycles d'amplification, chacun défini par les trois étapes suivantes :
- dénaturation de l'ADN 94°C pendant 1 min ;
- hybridation des amorces à l'ADN simple brin à 55°C pendant 2 min ;
- synthèse des nouveaux brins à 72°C.

La réaction s'effectue en présence d'un mélange de désoxynucléotides (dCTP, dGTP, dATP, dTTP, 200 μM final chacun) des deux amorces (0,5 μM) d'un μg d'ADN et 2,5 unités de Taq polymérase (CETUS). L'automatisation des étapes est assurée grâce au "cycler" Perkin Elmer Cetus.

Dans les conditions décrites, on obtient l'amplification d'une bande unique dont la taille, estimée à 250 pb après analyse sur un gel d'agarose à 2 %, correspond à celle attendue. L'utilisation de la sonde oligonucléotidique conforme à l'invention et définie par la formule IV ci-dessus permet de révéler par hybridation Southern la même bande et de vérifier ainsi la spécificité de la réaction.

Une expérience de dilution de l'ADN de tachyzoïte dans l'ADN humain permet de déterminer le seuil de détection de la technique comme étant l'équivalent de 10 tachyzoïtes.

La réaction est spécifique de l'ADN de *T. gondii* car dans la mesure où l'ADN humain, murin ou de hareng ne donne pas de produits d'amplification.

**EXEMPLE 8 : CLONAGE DE L'INSERT GPP5**

L'insert du clone d'ADNc P-30.8 sert pour le criblage d'une banque d'ADN génomique dans le vecteur lambda gt10. Un clone contenant un insert de 1600 paires de bases, qui correspond à un fragment de l'ADN génomique de *Toxoplasma gondii* et dénommé G42 par les Inventeurs est isolé.

Le sous clonage des fragments Pst I-Pst I conduit à la caractérisation et au séquençage du clone GPP5.

Ainsi que cela ressort de ce qui précède, l'invention ne se limite nullement à ceux de ses modes de mise en oeuvre, de réalisation et d'applications qui viennent d'être décrits de façon plus explicite ; elle en embrasse, au contraire, toutes les variantes qui peuvent venir à l'esprit du technicien en la matière, sans s'écarter du cadre, ni de la portée de la présente invention.

**Revendications**

1°) Séquence d'acide nucléique, caractérisée en ce qu'elle présente la séquence en bases nucléotidiques (I) suivante :

```
1          10          20          30          40          50
AAGGGGAGACCGGTGGACGGTCCAGGTCTTCGTGGAATGCTCGTCTTGCGCGCACCGGC

60          70          80          90          100         110
CGAAATTTCCGGTTCTACGACCCGGCGAAAGTGAAGAGCCTCGTGGTCACGGACTTCAG

120         130         140         150         160
TCGCCTCATGCTCCGCAAGGCCCTGGAGAAGAAAGAAGCCCTCAAG          (I),
```

et en ce qu'elle code pour une protéine reconnue par les anticorps anti-P30.

2°) Séquence d'acide nucléique, caractérisée en ce qu'elle comprend au moins un fragment de la séquence de formule (I) ci-dessus et en ce qu'elle présente la séquence en bases nucléotidiques de formule (II) ci-après :

```
       10          20          30          40          50          60
GTATCAGACCCTCGTCAAAAGTATCGTTCTTCAGCTGCGATAGAAGCGTCGCAGTGACGT

       70          80          90          100         110         120
CAAGGAGCACAGTGGCCAAGCTATACACACAGTCGTACCTTAAGTGACGATGCATTTTCT

       130         140         150         160         170         180
CGAGTTTCTCGCCAACACTCCGGTTGCTTGACAAGGGGAGACCGGTGGACGGTCCAGGTC

       190         200         210         220         230         240
TTCGTGGAATGCTCGTCTTGCGCGTAAGCAAGCGATCGACGACGGGCAACAAGAGGAACG

       250         260         270         280         290         300
AGGTCAGCTTCTTCAGTGCCGTTCAATCGGTATACGTCCAGCTGGGTCGCTGGAGTCGAG

       310         320         330         340         350         360
ACGCGGTTCGCAAGAGAGCGGACTGCGTGCATCTGTTGGGTTCGACAGTCGTGTGCTTCG
```

# EP 0 353 111 A1

370    380    390    400
ù      ù      ù      ù
ATGTCGAAAGGGGCAGCTGCGAGTCGGGGAGTTTCGTCGACCTGCAG

(II)

3°) Oligonucléotide, caractérisé en ce qu'il est constitué par un fragment d'une séquence nucléotidique selon la revendication 1 ou la revendication 2.

4°) Oligonucléotide selon la revendication 3, caractérisé en ce qu'il présente la séquence de formule III suivant :

5' CAGCTGCGATAGAAGCGTCGCAGTG    (III)

5°) Oligonucléotide selon la revendication 3, caractérisé en ce qu'il présente la séquence de formule IV suivante :

5' GGGGAGACCGGTGGACGGTCCAGGTC    (IV)

6°) Oligonucléotide selon la revendication 3, caractérisé en ce qu'il présente la séquence de formule (V) suivante :

5' GCAGGTCGACCCAGCGACCTCAGC    (V)

7°) Oligonucléotide selon l'une quelconque des revendications 3 à 6, caractérisé en ce qu'il est obtenu par synthèse.

8°) Paires d'amorces pour la synthèse d'un acide nucléique de *Toxoplasma gondii*, caractérisées en ce que chaque amorce comprend une séquence ou un fragment de séquence nucléotidique selon l'une quelconque des revendications 1 à 6.

9°) Paires d'amorces selon la revendication 8, caractérisées en ce qu'elles peuvent avantageusement être marquées par tout moyen approprié.

10°) Paire d'amorces selon la revendication 8 ou la revendication 9, caractérisée en ce qu'elle est constituée par un oligonucléotide de formule III selon la revendication 4 apparié à un oligonucléotide de formule V selon la revendication 6, pour la synthese dudit acide nucléique.

11°) Sondes nucléotidiques, caractérisées en ce qu'elles comprennent une séquence nucléotidique ou un fragment de celle-ci selon l'une quelconque des revendications 1 à 6, éventuellement marquée à l'aide d'un marqueur tel qu'un isotope radioactif, une enzyme appropriée, un fluorochrome ou par toute autre modification chimique ou un anticorps.

12°) Sonde selon la revendication 11, caractérisée en ce qu'elle est constituée par un oligonucléotide de formule IV selon la revendication 5.

13°) Protéine et/ou un fragment de celle-ci présentant des propriétés antigéniques, caractérisée en ce qu'elle est reconnue par les anticorps anti-P30 et en ce que sa séquence en acides aminés (VI), déduite de la séquence (I) selon la revendication I, est la suivante :

Gly$^1$-Glu-Thr-Gly-Gly-Arg-Ser-Arg-Ser-Ser$^{10}$-Trp-Asn-Ala-Arg-Leu-Ala-Arg-Thr-Gly-Arg$^{20}$-Asn-Phe-Arg-Phe-Tyr-Asp-Pro-Ala-Lys-Val$^{30}$-Lys-Ser-Leu-Val-Val$^{35}$-Thr-Asp-Phe-Ser-Arg$^{40}$-Leu-Met-Leu-Arg-Lys-Ala-Leu-Glu-Lys-Lys$^{50}$-Glu-Ala-Leu-Arg    (VI)

14°) Peptide, caractérisé en ce qu'il présente la séquence (VII) suivante en acides aminés :

Thr-Asp-Phe-Ser-Arg-Leu-Met-Leu-Arg-Lys-Ala-Leu-Glu-Lys-Lys-Glu-Ala-Leu-Arg,    (VII)

en ce qu'il est reconnu par les anticorps anti-P30 et en ce qu'il correspond à la fraction C-terminale de la protéine de formule VI selon la revendication 13.

15°) Peptide selon la revendication 14, caractérisé en ce que l'acide aminé N-terminal est une acétyl-cystéine et ledit peptide présente la séquence (VIII) suivante en acides aminés :

AcétylCys-Asp-Phe-Ser-Arg-Leu-Met-Leu-Arg-Lys-Ala-Leu-Glu-Lys-Lys-Glu-Ala-Leu-Arg.    (VIII)

16°) Conjugué présentant des propriétés antigé niques, caractérisé en ce qu'il est constitué par un peptide selon la revendication 14 ou la revendication 15, couplé par covalence à une protéine de transport.

17°) Conjugué selon la revendication 16, caractérisé en ce que la protéine de transport est constituée par de l'albumine bovine.

18°) Protéine et/ou un fragment de celle-ci présentant des propriétés antigèniques, caractérisée en ce qu'elle est reconnue par les anticorps anti-P30 et en ce que sa séquence en acides aminés (IX), déduite de la séquence II selon la revendication 2 est la suivante :

Glu-Gly-Glu-Thr-Gly-Gly-Arg-Ser-Arg-Ser-Ser-Trp-Asn-Ala-Arg-Leu-Ala-Arg-Lys-Gln-Ala-Ile-Asp-Asp-Gly-Gln-Gln-Glu-Glu-Arg-Gly-Gln-Leu-Leu-Gln-Cys-Arg-Ser-Ile-Gly-Ile-Arg-Pro-Ala-Gly-Ser-Leu-Glu-Ser-Arg-Arg-Gly-Ser-Gln-Glu-Ser-Gly-Leu-ArgAla-Ser-Val-Gly-Phe-Asp-Ser-Arg-Val-Leu-Arg-Cys-Arg-Lys-Gly-Gln-Leu-Arg-Val-Gly-Glu-Phe-Arg-Arg-Pro-Ala.    (IX)

19°) Produit d'expression de la protéine selon la revendication 13, caractérisé en ce qu'il est exprimé par un vecteur d'expression recombinant dans lequel a été insérée la séquence d'ADN de formule I et en ce qu'il a un poids moléculaire de 120 KDa.

20°) Produit d'expression selon la revendication 19, caractérisé en ce qu'il comprend un fragment peptidique de 6 KDa qui correspond à une partie d'une protéine antigénique de *Toxoplasma gondii*, est identique à la protéine de formule (VI) selon la revendication 13 et porte au moins un épitope reconnu par les anticorps anti-P30.

21°) Plasmide recombinant, caractérisé en ce qu'il comporte un insert d'ADN de formule I selon la revendication 1.

22°) Plasmide recombinant selon la revendication 21, caractérisé en ce qu'il est déposé auprès de la Collection Nationale de Cultures de Micro-organismes tenue par l'INSTITUT PASTEUR, en date du 24 juin 1988 sous le n° I-773.

23°) Plasmide recombinant, caractérisé en ce qu'il comporte un insert d'ADN contenant le fragment d'ADN de formule II selon la revendication 2.

24°) Plasmide recombinant selon la revendication 23, caractérisé en ce qu'il est déposé auprès de la Collection Nationale de Cultures de Micro-organismes tenue par l'INSTITUT PASTEUR, en data du 22 juin 1989 sous le n° I-876 .

25°) Produits de diagnostic de la toxoplasmose, caractérisés en ce qu'ils contiennent en tant que constituants actifs, une protéine de formule VI selon la revendication 13 et/ou une protéine de formule IX selon la revendication 18 et/ou un peptide de formule VII selon la revendication 14 ou VIII selon la revendication 15 et/ou un conjugué selon l'une quelconque des revendications 16 ou 17 reconnu(s) par les anticorps anti-P30.

26°) Produits de diagnostic de la toxoplasmose, caractérisés en ce qu'ils contiennent comme constituants actifs, une séquence d'acide nucléique et/ou un fragment de celle-ci selon l'une quelconque des revendications 1 à 6.

27°) Vaccins propres à procurer une protection significative contre la toxoplasmose, caractérisés en ce qu'ils contiennent en tant que constituant actif une protéine et/ou un peptide et/ou un conjugué selon l'une quelconque des revendications 13 à 18, éventuellement associé à au moins un véhicule pharmaceutique approprié.

28°) Procédé de détection et/ou de surveillance de la toxoplasmose, notamment de la toxoplasmose aiguë et de la toxoplasmose congénitale, caractérisée en ce qu'elle met en oeuvre au moins un produit de diagnostic selon l'une quelconque des revendications 25 ou 26.

29°) Procédé de détection selon la revendication 28, caractérisé en ce que l'on met en contact un produit de diagnostic selon la revendication 25, ci-après dé nommés fractions antigéniques, avec un échantillon biologique à tester, déterminant ainsi la liaison desdites fractions antigéniques avec des anticorps anti-P30, dans le cas où ceux-ci sont présents dans l'échantillon biologique à tester, et en ce que ledit complexe est révélé de manière appropriée.

30°) Procédé de détection selon la revendication 28, caractérisé en ce que l'on met en contact des produits de diagnostic représentés par une sonde selon la revendication 11, avec un échantillon biologique traité de manière appropriée, de façon à permettre l'accès de leurs acides nucléiques à la sonde et réalisation d'une hybridation effective lorsque les toxoplasmes recherchés sont présents, élimination de toute sonde non hybridée et détection appropriée des sondes d'ADN retenues par hybridation.

31°) Procédé de détection selon la revendication 30, caractérisé en ce que préalablement à l'hybridation, on met en contact, dans des conditions appropriées, l'acide nucléique à détecter avec au moins une paire d'amorces selon l'une quelconque des revendications 8 à 10, pour amplifier au moins un fragment dudit acide nucléique.

32°) Kit, ou coffret pour la détection de *Toxoplasma gondii* dans des échantillons biologiques, caractérisé en ce qu'il comprend :

- une quantité appropriée de protéine et/ou de peptide et/ou de conjugué selon l'une quelconque des revendications 13 à 18, le cas échéant un témoin positif, un témoin négatif et tous autres réactifs appropriés.

33°) Kit ou coffret pour la détection de *Toxoplasma gondii* dans des échantillons biologiques, caractérisé en ce qu'il comprend une quantité appropriée de sonde selon la revendication 11, éventuellement associée à une quantité appropriée d'au moins une paire d'amorces convenables selon l'une quelconque des revendications 8 à 10 et le cas échéant des préparations témoins d'acides nucléiques de *Toxoplasma gondii* et tous autres réactifs appropriés.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,O | JOURNAL OF CELLULAR BIOCHEMISTRY, supplément 10A, abstracts of the 15th annual meetings - UCLA SYMPOSIA ON MOLECULAR & CELLULAR BIOLOGY", 20 janvier-15 février 1986, page 152, résumé no. C106, Alan R. Liss, Inc., New York, US; J.B. PRINCE et al.: "Characterization and cDNA cloning of a protective antigen from Toxoplasma gondii" * Résumé * | 1-33 | C 12 N 15/00<br>A 61 K 39/002<br>G 01 N 33/569<br>C 12 Q 1/68<br>C 07 K 7/08<br>C 07 K 15/00 |
| X,O | JOURNAL OF CELLULAR BIOCHEMISTRY, supplément 10A, abstracts of the 15th annual meetings - "UCLA SYMPOSIA ON MOLECULAR & CELLULAR BIOLOGY", 20 janvier - 15 février 1986, page 145, résumé no. C85, Alan R. Liss, Inc., New York, US; J.L. BURG et al.: "Molecular cloning and analysis of genes encoding Toxoplasma gondii antigens" * Résumé * | 1-33 | |
| X,O | CHEMICAL ABSTRACTS, vol. 109, no. 19, 7 novembre 1988, page 188, résumé no. 164664g, Columbus, Ohio, US; J.C. BOOTHROYD et al.: "Antigen and tubulin genes of Toxoplasma gondii", & UCLA SYMP. MOL. CELL. BIOL., NEW SER. 1987, 42(Mol. Strategies Parasit Invasion), 237-50 * Résumé *  ---    -/- | 1-33 | **DOMAINES TECHNIQUES RECHERCHES (Int. Cl.4)**<br><br>C 12 N<br>A 61 K<br>G 01 N |

Le présent rapport a été établi pour toutes les revendications

| Lieu de la recherche | Date d'achèvement de la recherche | Examinateur |
|---|---|---|
| LA HAYE | 29-09-1989 | SKELLY J.M. |

EPO FORM 1503 03.82 (P0402)

Office européen
des brevets

**RAPPORT DE RECHERCHE EUROPEENNE**

Numero de la demande

EP 89 40 1783

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,X | THE JOURNAL OF IMMUNOLOGY, vol. 130, no. 5, mai 1983, pages 2407-2412, The American Association of Immunologists, US; L.H. KASPER et al.: "Purification of a major membrane protein of Toxoplasma gondii by immunoabsorption with a monoclonal antibody" * En entier * | 13-20, 25,27- 29,32 | |
| X | THE JOURNAL OF IMMUNOLOGY, vol. 134, no. 5, mai 1985, pages 3426-3431, The American Association of Immunologists, US; L.H. KASPER et al.: "An unexpected response to vaccination with a purified major membrane tachyzoite antigen (P30) of Toxoplasma gondii" * En entier * | 27 | |
| P,X | THE JOURNAL OF IMMUNOLOGY, vol. 141, no. 10, 15 novembre 1988, pages 3584-3591, The American Association of Immunologists, US; J.L. BURG et al.: "Molecular analysis of the gene encoding the major surface antigen of Toxoplasma gondii" * En entier * | 1-33 | DOMAINES TECHNIQUES RECHERCHES (Int. Cl.4) |
| P,X | BIOLOGICAL ABSTRACTS, vol. 87, résumé no. 126646; J.B. PRINCE et al.: "Cloning of complementary DNA encoding A 28-kilodalton antigen of Toxoplasma gondii", & MOL. BIOCHEM. PARASITOL. 34(1), 1989, 3-14 * Résumé * | 1-33 | |

Le présent rapport a été établi pour toutes les revendications

| Lieu de la recherche | Date d'achèvement de la recherche | Examinateur |
|---|---|---|
| LA HAYE | 29-09-1989 | SKELLY J.M. |